# EUROPEAN PATENT APPLICATION

(11) **EP 1 946 754 A1**
(43) Date of publication of application: **23.07.2008**
(21) Application number: 06822095.3
(22) Date of filing: 24.10.2006
(51) Int. Cl.: A61K 31/202, A61P 1/00, A61P 1/04, A61P 17/00

(54) **THERAPEUTIC AGENT FOR ULCERATIVE COLITIS AND/OR CROHN'S DISEASE**

(30) Priority: 24.10.2005 JP 2005308522
(71) Applicant: Yamamoto, Keiko, Tokyo 165-0032 (JP); Yamada, Sachiko, Hachioji-shi, Tokyo 193-0845 (JP); Okazaki, Yasushi, Nishitokyo-shi, Tokyo 1880001 (JP); Nishii, Yasuho, Setagaya-ku, Tokyo 1580095 (JP)
(72) Inventor: Yamamoto, Keiko, Tokyo 165-0032 (JP); Yamada, Sachiko, Hachioji-shi, Tokyo 193-0845 (JP); Okazaki, Yasushi, Nishitokyo-shi, Tokyo 1880001 (JP); Nishii, Yasuho, Setagaya-ku, Tokyo 1580095 (JP)
(74) Representative: Lillegraven, Rita
(86) International application number: PCT/JP2006/321115
(87) International publication number: WO 2007/049586

(57) **Abstract**

Disclosed is an agent for preventing or treating ulcerative colitis, Crohn's disease, and/or Behcet's disease. A therapeutic agent for ulcerative colitis, Crohn's disease, and/or Behcet's disease, comprising a polyunsaturated fatty acid derivative represented by the following formula (1) as an active ingredient.

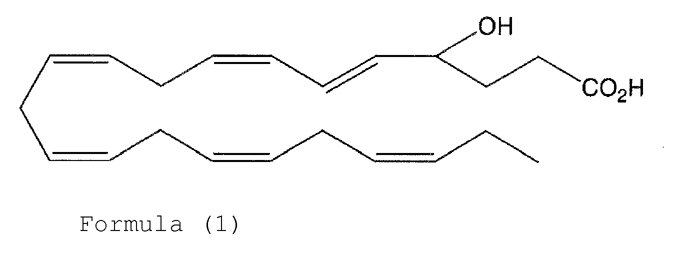

## Description

### TECHNICAL FIELD

The present invention relates to an agent for preventing or treating ulcerative colitis, Crohn's disease, and/or Behcet's disease.

### BACKGROUND ART

Among the inflammatory bowel diseases, ulcerative colitis and Crohn's disease in particular have a high frequency of occurrence and are refractory, protracted diseases that are difficult to treat clinically. Ulcerative colitis and Crohn's disease are chronic inflammatory diseases in which erosions and ulcers are formed in the intestinal mucous membranes and which cause diarrhea-like symptoms accompanied by bloody bowel discharge. These diseases have been designated as specified refractory diseases at the Ministry of Health, Labor, and Welfare of Japan. Ulcerative colitis is treated by the administration of salazosulfapyridine and prednisolone, with the drug dose being adjusted in correspondence to the symptoms. In the case of Crohn's disease, the small intestine type of the disease is treated using adrenocortical hormones (steroids), while the colonic type of the disease is treated using salazosulfapyridine (see Non-Patent Documents 1, 2 and 3). Behcet's disease, on the other hand, afflicts fewer people than ulcerative colitis and Crohn's disease; it is a refractory disease and is treated with the same drug treatment as for ulcerative colitis and Crohn's disease (see Non-Patent Document 4). However, side effects occur frequently with salazosulfapyridine due to its metabolic product, sulfapyridine, and there are also side effects associated with the steroids, and as a consequence a significant number of cases encounter problems in administration of these drugs in clinical settings (see Non-Patent Document 5).

Docosahexaenoic acid (DHA), on the other hand, has long been used as a health food and is a compound with very few reports of side effects. 4(S)-OH-DHA, which is an optically active substance, is known to be a metabolite of naturally occurring DHA (see Non-Patent Documents 6, 7 and 8). However, nothing was known about the efficacy of the polyunsaturated fatty acid derivative of the following formula (1) on inflammatory bowel diseases, such as ulcerative colitis and Crohn's disease.

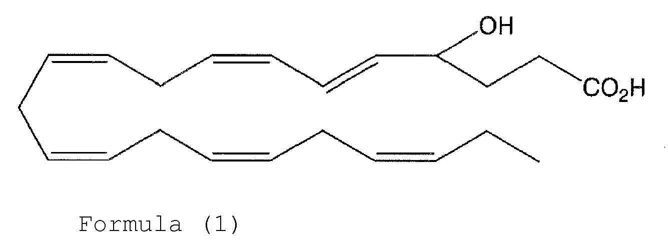

Non-Patent Document 1: Folia Pharmacol. Jpn., Volume 122, pages 309 to 316 (2003)(page 309, right column, lines 3 to 11) Non-Patent Document 2: BIO Clinica, Volume 12, 334-338 (1997) (page 334, left column, line 12 to page 337, right column, line 1)
Non-Patent Document 3: Journal of the Japan Society of Coloproctology, Volume 48, 1144-1152 (1995) (page 1144, left column, line 6 to page 1147, right column, line 13)
Non-Patent Document 4: Guidebook to Behcet's Disease, Takahide MATSUDA, Nihon Igakukan
Non-Patent Document 5: Diagnosis and Treatment, Volume 85, 933-938 (1997) (page 933, right column, lines 1 to 20)
Non-Patent Document 6: Hong, S., Gronert, K., Devchand, P.R., Moussignac, R.L., Serhan, C.N., J. Biol. Chem., 2003, 278, 14677-14687 (page 14680, left column, lines 46 to 65)
Non-Patent Document 7: Corey, E.J., Shih, C., Cashman, J.R., Proc. Natl. Acad. Sci. USA, 1983, 80, 3581-3584 (page 3583, left column, lines 36 to 61)
Non-Patent Document 8: Fischer, S., v. Schacky C., Siess, W., Strasser, T., Weber, P.C., Biochem. Biophys. Res. Commun., 1984, 120, 907-918 (page 911, lines 7-16)

### DISCLOSURE OF THE INVENTION

An object of the present invention is to provide a safe, side effect-free therapeutic agent for ulcerative colitis.

Another object of the present invention is to provide a safe, side effect-free therapeutic agent for Crohn's disease.

Another object of the present invention is to provide a safe, side effect-free therapeutic agent for Beçhet's disease.

### SUMMARY OF THE INVENTION

As a result of investigations in order to achieve the aforementioned objects, the present inventors discovered that the compound represented by the following formula (1), which is a derivative of a polyunsaturated fatty acid, has a therapeutic effect on ulcerative colitis, Crohn's disease, and/or Beçhet's disease.

The present invention is directed to a therapeutic agent for ulcerative colitis, comprising a polyunsaturated fatty acid derivative represented by the following formula (1) as an active ingredient.

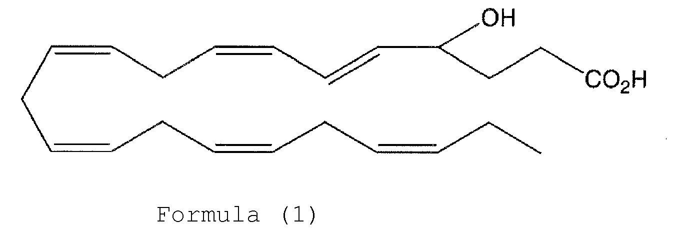

The present invention is further directed to a therapeutic agent for Crohn's disease, comprising the polyunsaturated fatty acid derivative represented by formula (1) above as an active ingredient.

The present invention is further directed to a therapeutic agent for Behcet's disease, comprising the polyunsaturated fatty acid derivative represented by formula (1) above as an active ingredient.

The present invention provides a very safe therapeutic agent for ulcerative colitis, Crohn's disease, and/or Behcet's disease.

### BRIEF DESCRIPTION OF THE DRAWINGS

Figure 1 is a graph showing the effect of YY-1 on the area of erosion in the mucous membrane of the colon in a rat model of DSS-induced ulcerative colitis;
Figure 2 is a graph showing the effect of YY-1 on colon contraction in a rat model of DSS-induced ulcerative colitis;
Figure 3 is a diagram of the experimental protocol in Example 2 for testing the activity of YY-1 in a mouse model of ulcerative colitis;
Figure 4 is a diagram showing the effect of YY-1 on body weight in a mouse model of DSS-induced ulcerative colitis;
Figure 5 is a graph showing the alleviation of symptoms by YY-1 in a mouse model of DSS-induced ulcerative colitis;
Figure 6 is a graph showing the influence of YY-1 on colon contraction in a mouse model of DSS-induced ulcerative colitis;
Figure 7 is a diagram showing the experimental protocol in Example 3 for testing the activity of YY-1 in a mouse model of ulcerative colitis;
Figure 8 is a diagram showing the influence of YY-1 on body weight in a mouse model of DSS-induced ulcerative colitis;
Figure 9 is a diagram showing the alleviation of symptoms by YY-1 in a mouse model of DSS-induced ulcerative colitis; and
Figure 10 is a graph showing the influence of YY-1 in a mouse model of DSS-induced ulcerative colitis.

### PREFERRED EMBODIMENTS OF THE INVENTION

The polyunsaturated fatty acid derivative of the invention is represented by the following formula (1).

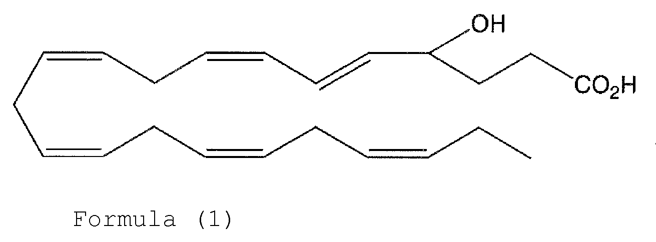

The polyunsaturated fatty acid derivative of the formula (1) (referred to as YY-1 herein for the sake of convenience) may be obtained by any methods known in the art, including, for example, chemical synthesis, fermentation and extraction from a natural source, followed by chromatography and concentration of the eluted fraction.

The dosage form of the therapeutic agent of the present invention is not particularly limited, but commonly used formulations may be employed, for example, an oral formulation (tablet, powdered drug, granule, powder, capsule, soft capsule, solution), a suppository, or an injectable.

The method of obtaining the therapeutic agent of the invention is not particularly limited, but may be formulated by usual methods using YY-1. In addition, a soft capsule formulation can also be made by adding or dissolving it in a natural oil, higher fatty acid oil, higher fatty acid monoglyceride, or a mixture thereof, and filling the resulting oil into the capsule. In this case, a gelatin-based soft capsule or a soft capsule based on another water-soluble polymer can be used. Such capsules may also include a microcapsule. In addition, YY-1 may be dissolved in an oil and solidified by usual methods to prepare a suppository. Also YY-1 may be dissolved in a solution with a surfactant to form an injectable.

In addition to YY-1, other pharmaceutically acceptable formulation excipients may conveniently be added to the therapeutic agent of the present invention by usual methods. Such excipients include, but not limited to, diluents, binders, disintegrants, lubricants, oxidation inhibitors, colorants, aggregation inhibitors, absorption promoters, dissolution enhancers for the active component, stabilizers, and so forth. The diluents include, but not limited to, sucrose, lactose, glucose, corn starch, mannitol, crystalline cellulose, calcium phosphate, calcium sulfate, and so forth. The disintegrant includes, but not limited to, starch, agar, calcium citrate, calcium carbonate, calcium bicarbonate, dextrin, crystalline cellulose, carboxymethyl cellulose, tragacanth, and so forth. The lubricant includes, but not limited to, talc, magnesium stearate, polyethylene glycol, silica, hardened plant oils, and so forth.

The binder includes, but not limited to, ethyl cellulose, methyl cellulose, hydroxypropyl methylcellulose, tragacanth, shellac, gelatin, gum arabic, polyvinylpyrrolidone, polyvinyl alcohol, polyacrylic acid, polymethacrylic acid, sorbitol, and so forth. The oxidation inhibitor includes, but not limited to, ascorbic acid, tocopherol, vitamin A, β-carotene, sodium bisulfite, sodium thiosulfate, sodium pyrosulfite, citric acid, and so forth.

The colorant is not particularly limited and, any colorants acceptable for addition to pharmaceutical products can be used. The aggregation inhibitor includes, but not limited to, stearic acid, talc, soft silicic anhydride, hydrated silicon dioxide, and so forth. The absorption promoter includes, but not limited to, higher alcohols, higher fatty acids, and surfactants such as glyceryl fatty acid esters. The dissolution enhancer for the active component includes, but not limited to, benzoic acid, sodium benzoate, ethyl para-hydroxybenzoate, and so forth.

Other pharmacologically active components may be present in the therapeutic agent of the invention. Such pharmacologically active components may include therapeutic agents for ulcerative colitis, such as mesalazine, salazosulfapyridine, prednisolone, and so forth, and therapeutic agents for irritable bowel diseases, such as Irricolon M, mepenzolate bromide, Trancolon P, and so forth.

The dosage of administration of the therapeutic agent of the present invention is generally 1 mg to 1 g/day/person, more desirably 5 to 500 mg/day/person, and even more desirably 30 to 200 mg/day/person.

### EXAMPLES

Examples and formulation examples are provided below in order to explain the present invention in greater detail; however, the present invention is not limited to only these examples and formulation examples.

### Reference Example 1

### Preparation of (5E,7Z,10Z,13Z,16Z,19Z)-4-hydroxy-5,7,10,13,16,19-docosahexaenoic acid (YY-1)

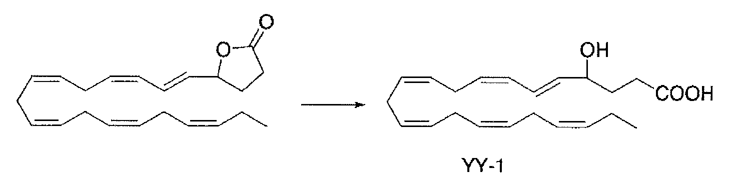

KOH (5% in H₂O/MeOH = 5/95, 2.85 mL) was added to 5-[(1E,3Z,6Z,9Z,12Z,15Z)-1,3,6,9,12,15-octadecahexaenyl] dihydro-2(3H)-furanone (93 mg, 0.285 mmol) and stirred for one hour while heating at 50°C under Ar. Ice was added to the reaction solution, neutralized with 2N aqueous HCl solution, and extracted with AcOEt. The organic layer was washed with water and dried over MgSO₄. The solvent was evaporated and the residue was purified by silica gel (10 g) column chromatography (40% AcOEt-hexane) to obtain YY-1 (91 mg, 92.8%).
¹H-NMR (400 MHz, CDCl₃) δ: 0.97 (3H, t, J=7.5 Hz, H-22), 1.88 (2H, m, H-3), 2.08 (2H, m, H-21), 2.48 (2H, t, J=7.3 Hz, H-2), 2.85 (6H, m, H-12, 15, 18), 2.97 (2H, t, J=6.6 Hz, H-9), 4.25 (1H, dd, J=12.6, 6.6 Hz, H-4), 5.38 (9H, m, H-8, 10, 11, 13, 14, 16, 17, 19, 20), 5.68 (1H, dd, J=15.1, 6.5 Hz, H-5), 6.01 (1H, t, J=11.0 Hz, H-7), 6.54 (1H, dd, J=15.1, 11.0 Hz, H-6); FABMS m/z (relative intensity) 345 (M⁺ + H, 1), 327 (9), 281 (5), 79 (100), 67 (88); UV (95% EtOH) λₘₐₓ 238 nm.

### Example 1

### Activity in a rat model of ulcerative colitis

The therapeutic effect of YY-1 was investigated with the 7-day repetitive intrarectal administration using a dextran sodium sulfate (DSS)-induced ulcerative colitis model in the rat *(*Cancer Research, 61, 2424-2428, 2001). To generate the rat model of DSS-induced ulcerative colitis, a 3% aqueous DSS solution was ingested ad libitum by SD rats (male, age: 7 weeks) purchased from Charles River Laboratories Japan Inc., and animals presenting bloody stools were used as the rat model of DSS-induced ulcerative colitis.

The negative control group received 0.5% aqueous CMC-Na solution (vehicle) by intrarectal administration, while the positive control group received 100 mg/kg/day 5-aminosalicylic acid (5-ASA, purchased from Aldrich Chemical Company) dissolved in 0.5% aqueous CMC-Na solution by intrarectal administration. For YY-1, the compound was combined with 0.5% aqueous CMC-Na solution by means of a vortex mixing and sonication to form a suspension, and was administered intrarectally at 20 mg/kg/day (low dose group), 100 mg/kg/day (intermediate dose group), and 500 mg/kg/day (high dose group). Each group contained 8 SD rats, and a 1% aqueous DSS solution was ingested ad libitum during the test period.

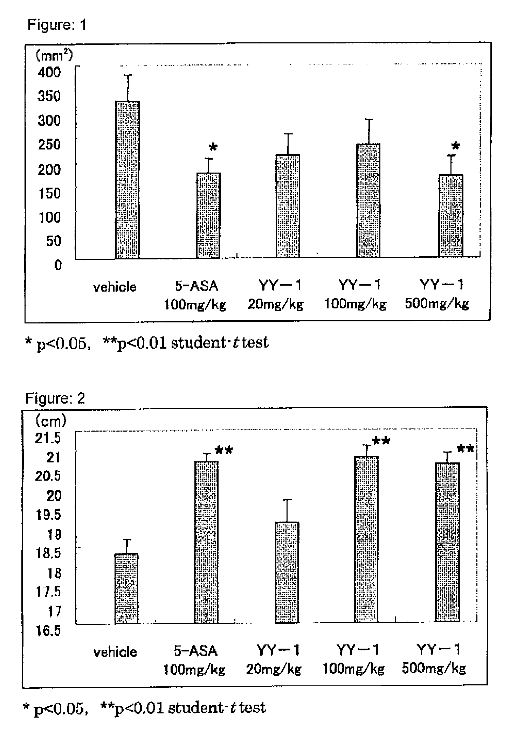

The results showed that the intrarectal administration of YY-1 at 500 mg/kg/day gave a significant reduction in the area of erosion in the mucous membrane of the colon, and that a significant improvement in colon contraction was observed for the intrarectal administration of 100 and 500 mg/kg/day (Figures 1 and 2).

The results of the hematological examinations are shown in table 1.

**Table 1: Results of hematological examinations**

| group | WBC (x 10²/µL) | RBC (x 10⁴/µL) | Hb (g/dL) | Ht (%) |
|---|---|---|---|---|
| normal group^{a)} | 91 ± 11 | 768 ± 8 | 15.4 ± 0.2 | 47.7 ± 1 |
| vehicle group^{a)} | 125 ± 21 | 499 ± 73 | 10.4 ± 1.4 | 33.3 ± 4.1 |
| 5-ASA 100 mg/kg/day, i.r. | 126 ± 20 | 633 ± 27 | 13.3 ± 0.4 | 41.7 ± 1.1 |
| YY-1 20 mg/kg/day, i.r. | 136 ± 34 | 519 ± 80 | 11 ± 1.5 | 35.5 ± 4.6 |
| YY-1 100 mg/kg/day, i.r. | 120 ± 13 | 605 ± 51 | 12.2 ± 0.9 | 41.5 ± 3.2 |
| YY-1 500 mg/kg/day, i.r. | 138 ± 14 | 670 ± 20 | 13.8 ± 0.3 | 45.7 ± 1.1 |

| | | | | |
|---|---|---|---|---|
| a): 0.5% CMC-Na, 2 mL/kg x 2/day, i.r. | | | | |

### Example 2

### Activity in a mouse model of ulcerative colitis

The therapeutic effect of YY-1 was investigated with the 7-day repetitive intrarectal administration using a dextran sodium sulfate (DSS)-induced ulcerative colitis model in the mouse. To generate the mouse model of DSS-induced ulcerative colitis, a 9.0% aqueous DSS solution was ingested ad libitum for 7 days by c57BL/6J (male, age: 8 weeks) purchased from CLEA Japan, Inc., and animals presenting body weight loss, bloody stool, and/or diarrhea were used as the mouse model of DSS-induced ulcerative colitis.

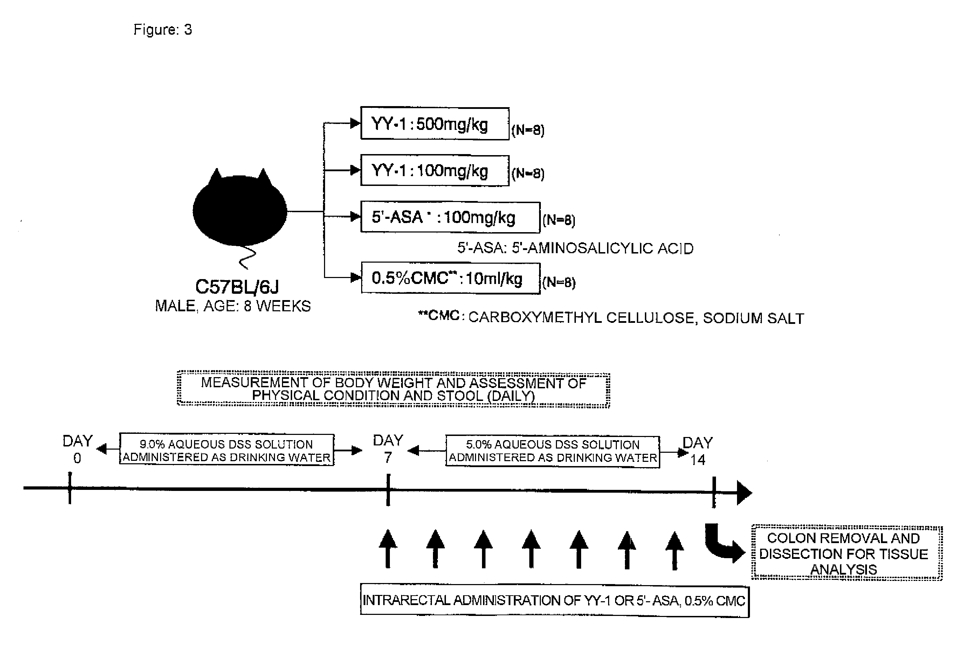

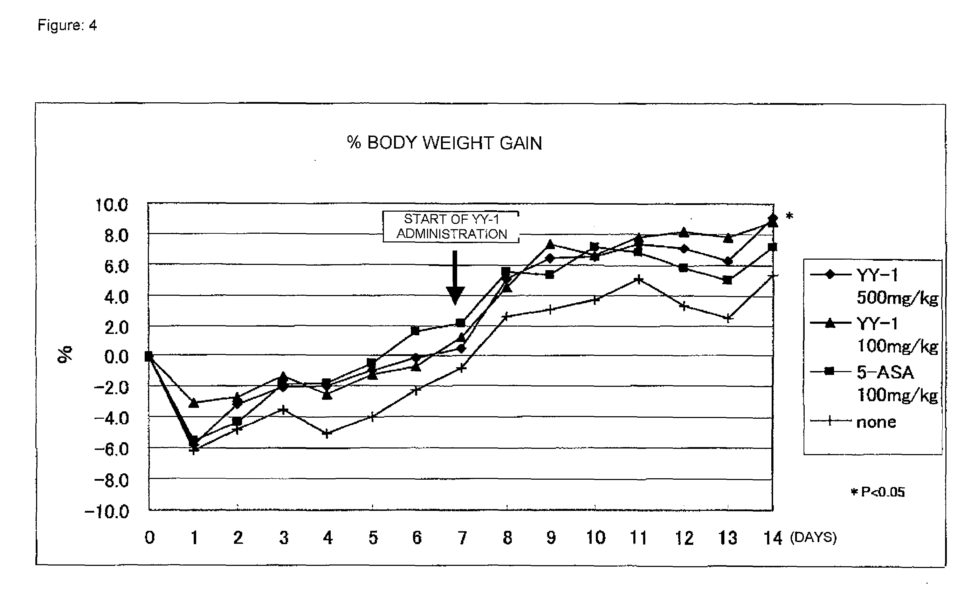

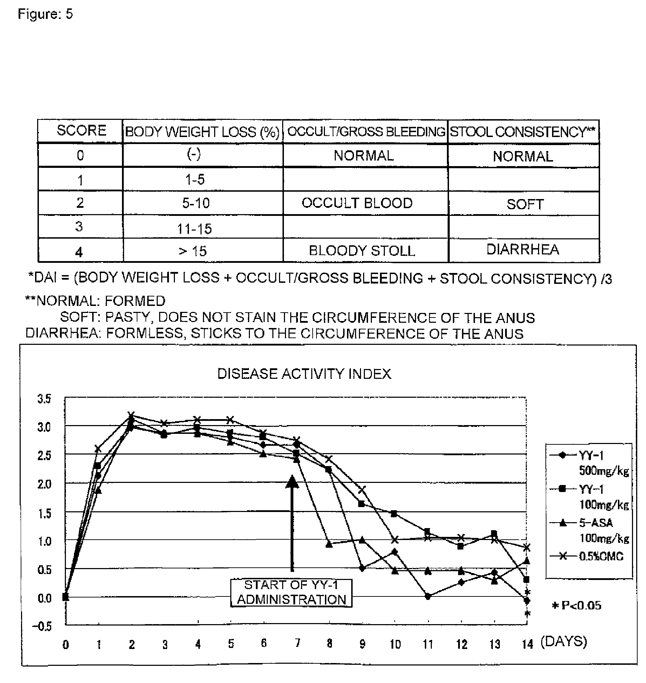

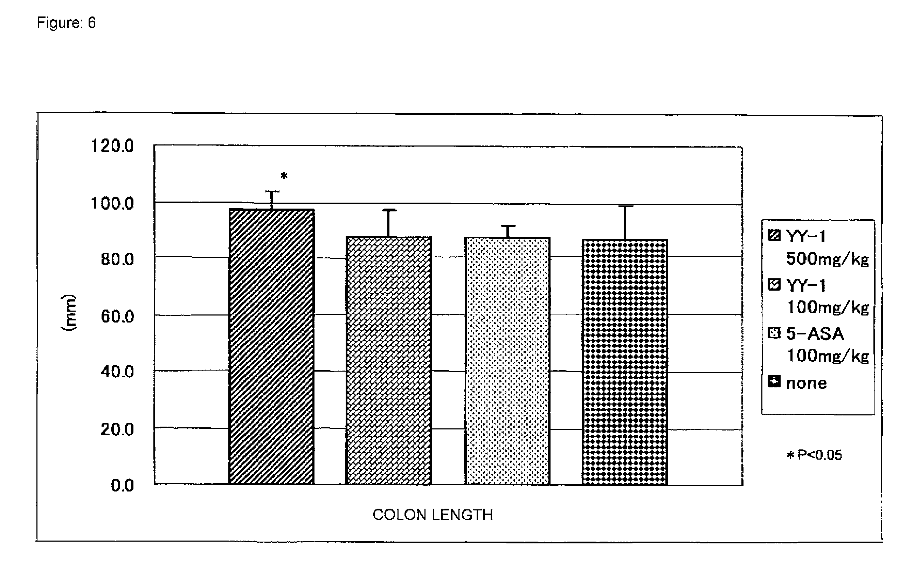

The negative control group received 0.5% aqueous CMC-Na solution by intrarectal administration, while the positive control group received 100 mg/kg/day 5-aminosalicylic acid (5-ASA, purchased from Aldrich Chemical Company) dissolved in 0.5% aqueous CMC-Na solution by intrarectal administration. For YY-1, the compound was combined with 0.5% aqueous CMC-Na solution by means of an ultrasound mixer under ice cooling to form a suspension, and was administered intrarectally at 100 mg/kg/day or 500 mg/kg/day. Each group contained 8 c57BL/6J mice, and a 5.0% aqueous DSS solution was ingested ad libitum during the test period (Figure 3).
The group receiving 100 mg/kg/day YY-1 and the group receiving 500 mg/kg/day YY-1 showed a significant improvement (P < 0.05) in body weight gain, symptom alleviation, and colon contraction (Figures 4, 5, and 6).

### Example 3

### Activity in a mouse model of ulcerative colitis

The therapeutic effect of YY-1 was investigated with the 7-day repetitive intrarectal administration using a dextran sodium sulfate (DSS)-induced ulcerative colitis model in the mouse. To generate the mouse model of DSS-induced ulcerative colitis, a 9.0% aqueous DSS solution was ingested ad libitum for 7 days by c57BL/6J (male, age: 8 weeks) purchased from CLEA Japan, Inc., and animals presenting body weight loss, bloody stool, and/or diarrhea were used as the mouse model of DSS-induced ulcerative colitis.

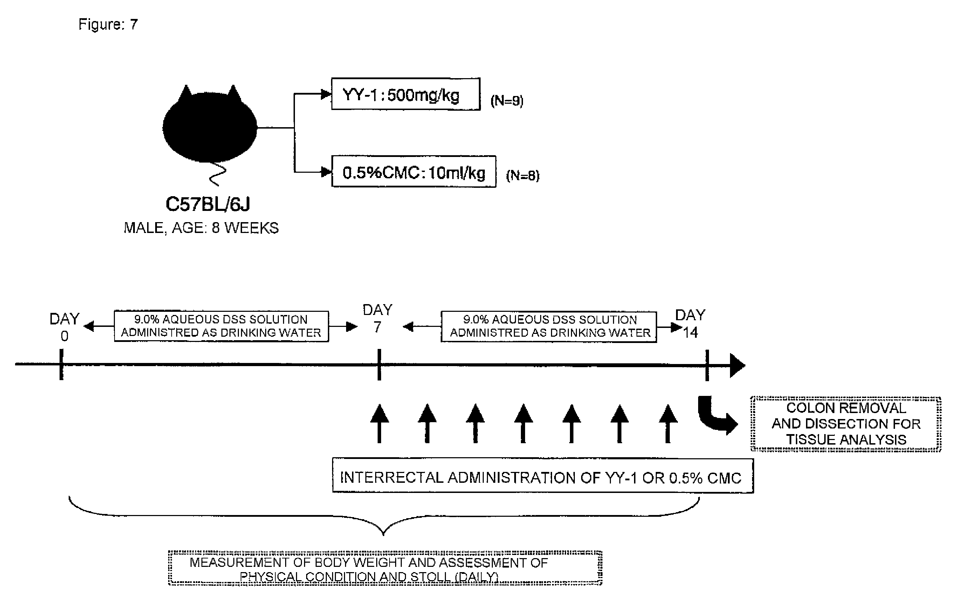

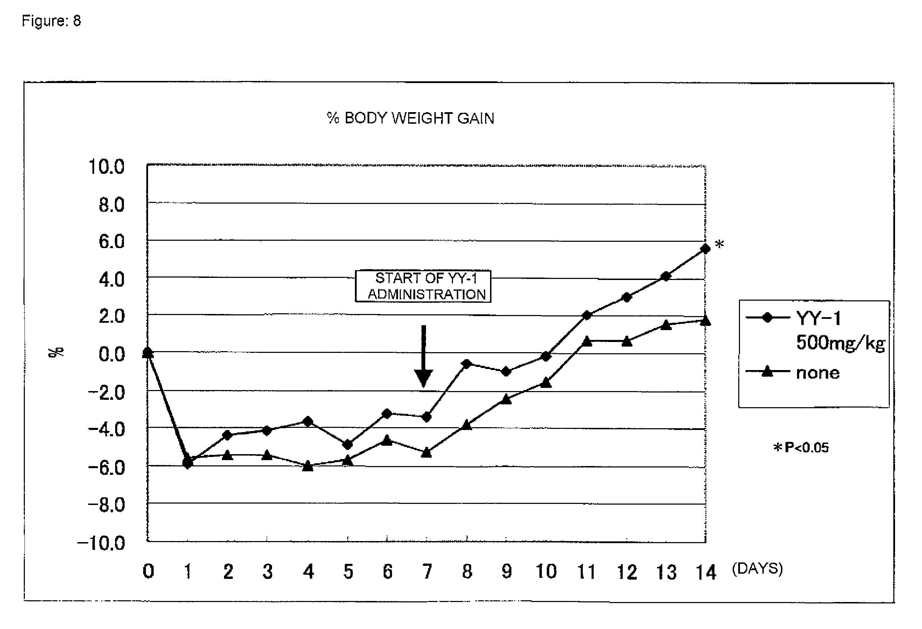

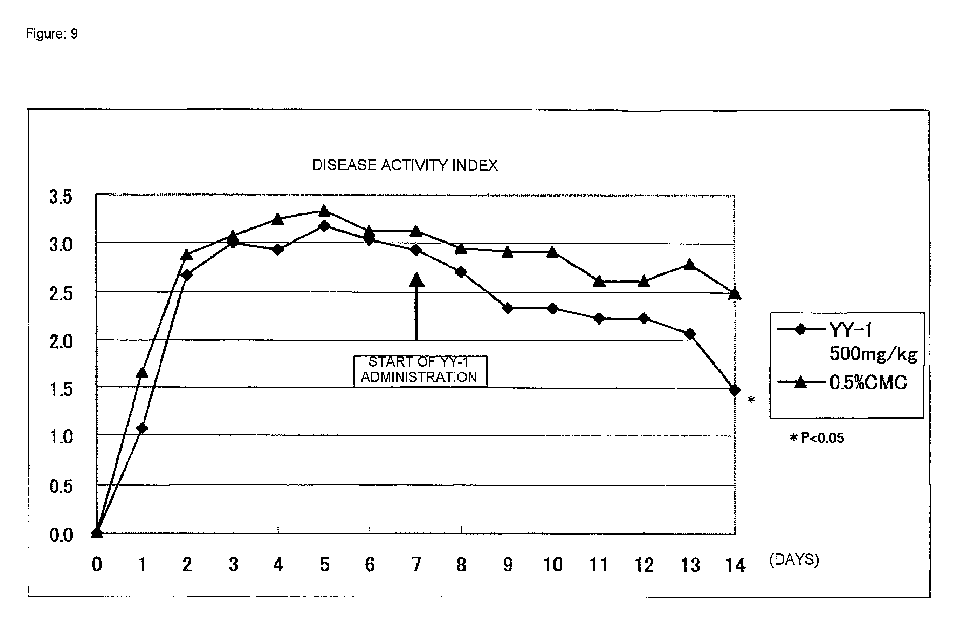

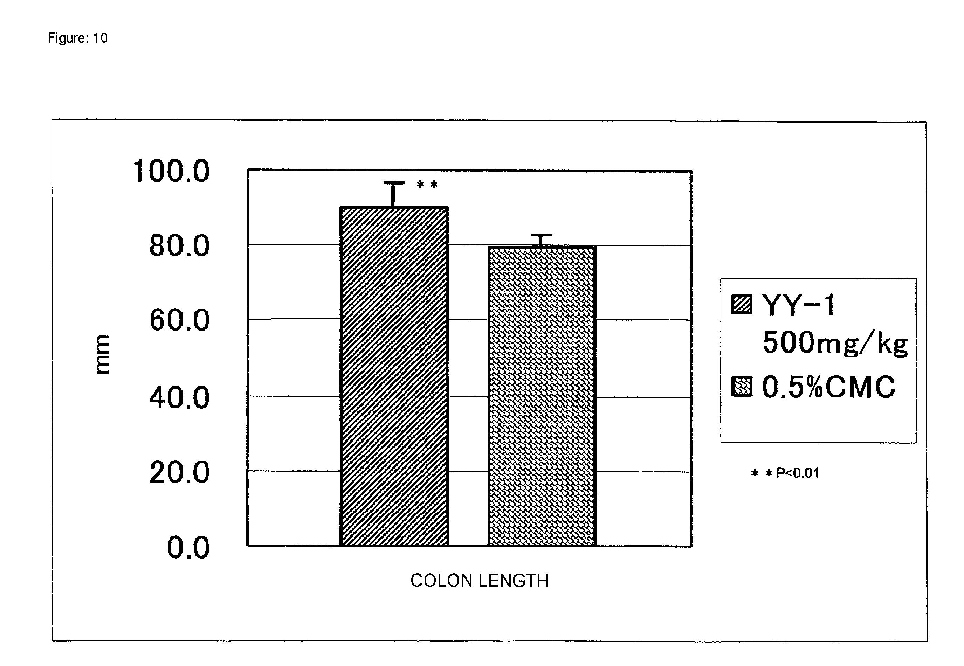

The negative control group received 0.5% aqueous CMC-Na solution by intrarectal administration. For YY-1, the compound was combined with 0.5% aqueous CMC-Na solution by means of an ultrasound mixer under ice cooling to prepare a suspension, and was administered intrarectally at 500 mg/kg/day. Each group contained 8 c57BL/6J mice, and a 9.0% aqueous DSS solution was ingested ad libitum during the test period (Figure 7).
The group receiving 500 mg/kg/day YY-1 showed a significant improvement in body weight gain, symptom alleviation, and colon contraction (body weight gain and symptom alleviation: P < 0.05, improvement in colon contraction: P < 0.01) (Figures 8, 9, and 10).

## Claims

1. A therapeutic agent for ulcerative colitis, comprising a polyunsaturated fatty acid derivative represented by the following formula (1) as an active ingredient.

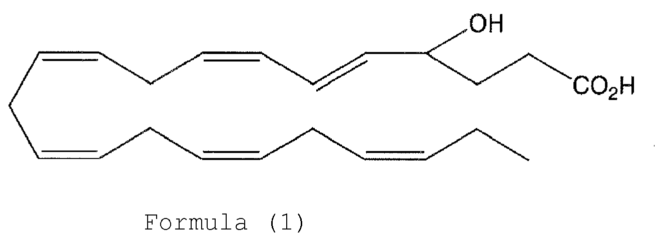

2. A therapeutic agent for Crohn's disease, comprising the polyunsaturated fatty acid derivative represented by formula (1) in claim 1 as an active ingredient.

3. A therapeutic agent for Behcet's disease, comprising the polyunsaturated fatty acid derivative represented by formula (1) in claim 1 as an active ingredient.
